# EUROPÄISCHE PATENTSCHRIFT

(11) **EP 2 114 855 B1**
(45) Veröffentlichungstag und Bekanntmachung des Hinweises auf die Patenterteilung: **27.07.2016**
(21) Anmeldenummer: 08707171.8
(22) Anmeldetag: 22.01.2008
(51) Int. Cl.: C07C 67/343, C07C 69/716

(54) **VERFAHREN ZUM HERSTELLEN VON DIHALOACETESSIGSÄUREALKYLESTERN**
METHOD FOR PRODUCING DIHALO ACETOACETIC ALKYL ESTERS
PROCEDE DE PRODUCTION D'ALKYLESTERS D'ACIDE DIHALOACETIQUE

(30) Priorität: 02.02.2007 DE 102007005296
(43) Veröffentlichungstag der Anmeldung: 11.11.2009
(73) Patentinhaber: Bayer Intellectual Property GmbH, 40789 Monheim am Rhein (DE)
(72) Erfinder: PAZENOK, Sergii, 42699 Solingen (DE); LUI, Norbert, 51519 Odenthal (DE)
(74) Vertreter: BIP Patents
(86) Internationale Anmeldenummer: PCT/EP2008/000438
(87) Internationale Veröffentlichungsnummer: WO 2008/092583

(56) Entgegenhaltungen:
- WO-A-2005/003077
- WO-A-2006/005612

## Beschreibung

Die Erfindung betrifft ein Verfahren zum Herstellen von Verbindungen der Formel (I) durch Umsetzung von α,α-Dihaloaminen der Formel (III) mit Carbonsäureestern der Formel (II) in Gegenwart von Basen.

Difluoroacetessigsäurealkylester sind wichtige Synthesebausteine zur Herstellung von Agrowirkstoffen, insbesondere zur Herstellung von Pyrazolylcarboxaniliden.

Aus Tetrahedron 2001, 57, 2689-2700 ist bekannt, dass man 4,4-Difluoroacetessigsäureester durch Umsetzung von Difluoroacetessigsäureethylester mit Ethylacetat in Gegenwart von Natriumhydrid (NaH) erhalten kann. Die Ausbeute dieser Reaktion ist mit 25 % sehr unbefriedigend. Außerdem ist in Tetrahedron 1996, 52, 119-130 beschrieben, dass sich 4,4-Difluoroacetessigsäureester durch die Reaktion von Difluoressigsäureethylester mit Bromessigsäureethylester in Gegenwart von Zn herstellen lässt.

WO-A-2005/003077 lehrt ein dreistufigen Verfahren zum Herstellen von Difluoracetessigsäurealkylestern ausgehend von Chlorodifluoroacetessigsäureestern durch Reduktion des Chloratoms mit Trialkoxyphosphinen (P(OAlk)₃), das auch als Perkow Reaktion bezeichnet wird.

WO-A-2006/005612 lehrt ein Verfahren zur Herstellung von 4,4-Difluor-3-oxo-buttersäureestern durch Umsetzung von 2,2-Difluor-N,N-dialkyl-acetamid mit Essigsäureestern in Gegenwart von Basen. Der 4,4-Difluor-3-oxo-buttersäurealkylester wird anschließend, wie in JACS, 73, 3684 (1951) beschrieben, mit Orthoameisensäuretrimethylester und Essigsäureanhydrid zum Ethyl-(2-ethoxymethylen)-4,4-difluoromethylacetoacetat umgesetzt, welches, gemäß der US-A-5,489,624, mit Methylhydrazin zum 3-Difluormethyl-1-methyl-4-pyrazolcarbonsäureethylester überführt werden kann. Die beschriebene Route beinhaltet zum einen eine Vielzahl von Reaktionsschritten, zum anderen ist das verwendete 2,2-Difluor-N,N-dialkyl-acetamid nicht kommerziell zugänglich und kann nur in geringen Ausbeuten von ca. 70 % durch Fluorierung von 2,2-Dichlor-N,N-dialkyl-acetamid erhalten werden.

Die im Stand der Technik vorbeschriebenen Verfahren weisen den Nachteil auf, dass die verwendeten Difluorcarbonsäurehalogenide, Halogenalkylcarbonsäureanhydride und Haloacrylester teuer sind, Korrosionsprobleme verursachen und/oder nur mit großem technischem Aufwand gereinigt werden können.

Der vorliegenden Erfindung liegt daher die Aufgabe zugrunde ein einfacheres und wirtschaftlicheres Verfahren zur Herstellung von Verbindungen der Formel zur Verfügung zu stellen.

Die Aufgabe wurde überraschenderweise gelöst durch ein Verfahren zur Herstellung von Verbindungen der Formel (I) 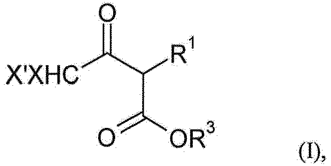 in der
- X: Fluor, Chlor oder CF₃ ist,
- X': Fluor, Chlor oder Brom ist,
- R¹: ausgewählt ist aus H, C₁₋₁₂-Alkyl-Resten, C₅₋₁₈-Aryl, Chlor, Brom und Fluor und
- R³: unabhängig von R¹ ausgewählt ist aus C₁₋₁₂-Alkyl-, C₅₋₁₈-Aryl- oder C₇₋₁₉-Arylalkyl-Resten,
durch Umsetzung von α,α-Dihalogenaminen der Formel (III) 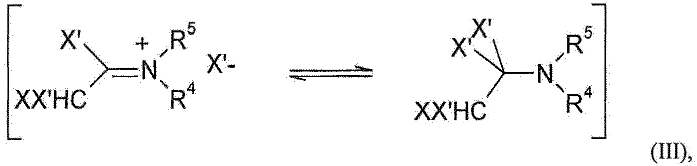 in welcher
R⁴ ausgewählt ist aus C₁₋₁₂-Alkyl-Resten, C₅₋₁₈-Aryl- oder C₇₋₁₉-Arylalkyl-Resten,
R⁵ unabhängig von R⁴ ausgewählt ist aus C₁₋₁₂-Alkyl-Resten, C₅₋₁₈-Aryl- oder C₇₋₁₉-Arylalkyl-Resten,
mit Carbonsäureestern der Formel (II) 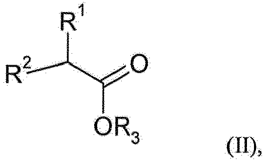 in welcher
- R²: ausgewählt ist aus Wasserstoff, C₁₋₁₂-Alkyl-Resten, C₅₋₁₈-Aryl, Chlor, Brom und Fluor.

In einer bevorzugten Ausfuhrungsform der vorliegenden Erfindung ist
- X: ausgewählt aus Fluor, Chlor oder CF₃;
- X': ausgewählt aus Fluor oder Chlor;
- R¹: ausgewählt aus C₁₋₄-Alkyl-Resten;
- R²: unabhängig von R¹ ausgewählt aus C₁₋₄-Alkyl-Resten;
- R³: unabhängig von R¹ ausgewählt aus C₁₋₄-Alkyl;
- R⁴: ausgewählt aus C₁₋₄-Alkyl-Resten;
- R⁵: unabhängig von R⁴ ausgewählt aus C₁₋₄-Alkyl-Resten.

In einer besonders bevorzugten Ausführungsform der vorliegenden Erfindung ist
- X: ausgewählt aus Fluor oder Chlor;
- X': Fluor;
- R¹: ausgewählt aus Wasserstoff oder Fluor;
- R²: Wasserstoff;
- R³: ausgewählt aus Methyl oder Ethyl;
- R⁴: ausgewählt aus Methyl oder Ethyl;
R⁵ ausgewählt aus Methyl oder Ethyl.
Weitere Ausführungsformen der vorliegenden Erfindung können den abhängigen Ansprüchen und der Beschreibung entnommen werden.

Das erfindungsgemäße Verfahren kann anhand des folgenden Schema (I) erläutert werden:

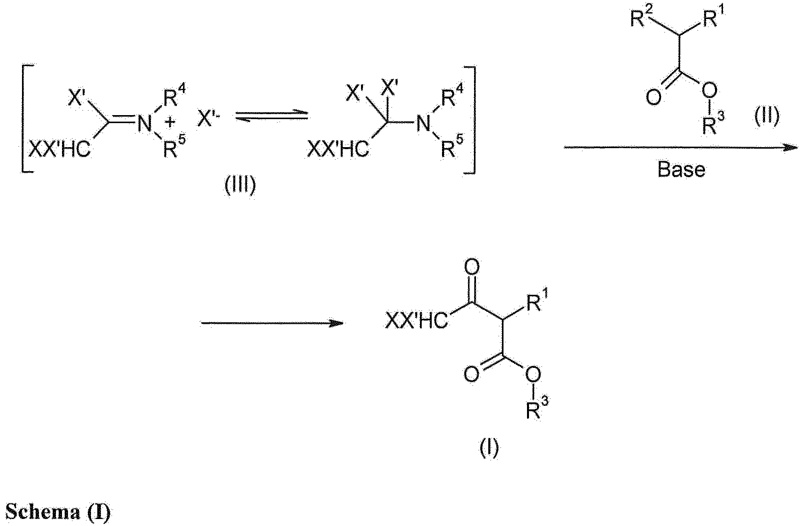

### Allgemeine Definitionen

Im Zusammenhang mit der vorliegenden Erfindung umfasst der Begriff Halogene (X), solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Reste können einfach oder mehrfach substituiert sein, wobei bei Mehrfachsubstitutionen die Substituenten gleich oder verschieden sein können.

Alkyl-Reste sind im Zusammenhang mit der vorliegenden Erfindung, soweit nicht abweichend definiert, lineare, verzweigte oder ringförmige Kohlenwasserstoff-Reste, die optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkyl-Reste optional durch weitere Gruppen substituiert sein, die ausgewählt sind aus -X, -OR', -SR', -NR'₂, -SiR'₃, -COOR', -CN und -CONR₂' Gruppen, wobei R' Wasserstoff oder eine C₁₋₄₋Alkyl-Gruppe sein kann.

Die Definition C₁₋C₁₂-Alkyl umfasst den größten hierin definierten Bereich für einen Alkyl-Rest. Im Einzelnen umfasst diese Definition beispielsweise die Bedeutungen Methyl, Ethyl, n-, isoPropyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Aryl-Reste sind im Zusammenhang mit der vorliegenden Erfindung, soweit nicht abweichend definiert, cyclische aromatische Kohlenwasserstoff-Reste, die ein, zwei oder mehrere Heteroatome, die ausgewählt sind aus O, N, P und S aufweisen können und optional durch weitere Gruppen substituiert sein können, die ausgewählt sind aus -X, -OR', -SR', -NR'₂, -SiR'₃, -COOR', -CN und -CONR₂' Gruppen, wobei R' Wasserstoff oder eine C₁₋₄₋Alkyl-Gruppe sein kann.

Die Definition C₅₋₁₈-Aryl umfasst den größten hierin definierten Bereich für einen Aryl-Rest mit 5 bis 18 Gerüstkohlenstoffatomen. Im Einzelnen umfasst diese Definition beispielsweise die Bedeutungen Cyclopentenyl, Phenyl, Cycloheptatrienyl, Cyclooctatetraenyl, Naphthyl und Anthracenyl.

Arylalkyl-Reste sind im Zusammenhang mit der vorliegenden Erfindung, soweit nicht abweichend definiert, cyclische aromatische Kohlenwasserstoff-Reste, die ein, zwei oder mehrere Heteroatome, die ausgewählt sind aus O, N, P und S aufweisen können und die wenigstens eine C₁₋₈-Alkyl-Seitenkette aufweisen, die optional mit einer weiteren Seitenkette einen vier-, fünf- oder sechsgliedrigen Ring bilden kann und optional durch weitere Gruppen substituiert sein kann, die ausgewählt sind aus -X, -OR', -SR', -NR'₂, -SiR'₃, -COOR', -CN und -CONR₂' Gruppen, wobei R' Wasserstoff oder eine C₁₋₄₋Alkyl-Gruppe sein kann.

Die Definition C₇₋₁₉-Arylalkyl-Rest umfasst den größten hierin definierten Bereich für einen Arylalkyl-Rest mit insgesamt 7 bis 19 Kohlenstoffatomen in Gerüst und Seitenkette. Im Einzelnen umfasst diese Definition beispielsweise die Bedeutungen Tolyl, *o*-Xylyl, *m*-Xylyl, *p*-Xylyl und Ethylphenyl.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

### Carbonsäureester

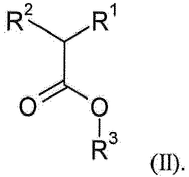

Bei dieser sind die Reste R² und R³ unabhängig voneinander ausgewählt aus H, C₁₋₁₂-Alkyl-, C₅₋₁₈-Aryl- oder C₇₋₁₉-Arylalkyl-Resten, vorzugsweise aus C₂₋₈-Alkyl-Resten, besonders bevorzugt aus C₃₋₆-Alkyl-Resten.

Beispiele geeigneter Carbonsäureester sind Essigsäuremethylester, Essigsäureethylester, Fluoressigsäureethylester, Bromessigsäureethylester, Propionsäureethylester, Phenylessigsäure-ethylester, Benzoesäureethylester.

Gemäß der vorliegenden Erfindung sind Essigsäureester bevorzugt, wobei Ethylacetat besonders bevorzugt ist.

### α,α-Dihalogenamine

Die gemäß der vorliegenden Erfindung verwendeten α,α-Dihalogenamine sind Verbindungen gemäß der allgemeinen Formel (III) 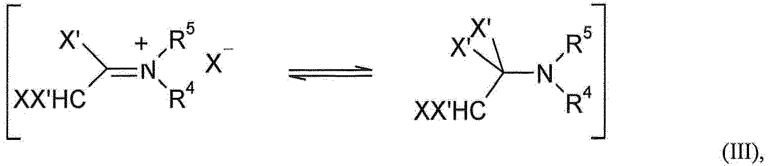 in welcher
R⁴ ausgewählt ist aus C₁₋₁₂-Alkyl-, C₅₋₁₈-Aryl- oder C₇₋₁₉-Arylalkyl-Resten, vorzugsweise aus C₂₋₈-Alkyl-Resten, besonders bevorzugt aus C₃₋₆-Alkyl-Resten;
R⁵ unabhängig von R⁴ ausgewählt ist aus C₁₋₁₂-Alkyl-, C₅₋₁₈-Aryl- oder C₇₋₁₉-Arylalkyl-Resten, vorzugsweise aus C₂₋₈-Alkyl-Resten, besonders bevorzugt aus C₃₋₆-Alkyl-Resten;
   - X: CF₃, Fluor oder Chlor ist, wobei Fluor bevorzugt ist und
   - X': Fluor, Chlor oder Brom, wobei Fluor bevorzugt ist.

Die Verbindungen sind gemäß Petrov et al. in Journal of Fluorine Chemistry 109 (2001) 25-31 und Dmowski et al. in Chemistry of Organic Fluorine Compounds II, A Critical Review, ACS, Washington DC (1995) 263 durch Umsetzung von fluorierten/halogenierten Alkenen mit sekundären Aminen zugänglich und werden kommerziell beispielsweise durch DuPont vertrieben.

Die gemäß der vorliegenden Erfindung bevorzugt eingesetzten α-Halogenamine sind beispielsweise ausgewählt aus der Gruppe bestehend aus 1,1,2,2-Tetrafluorethyl-N,N-dimethylamin, 1,1,2,2-Tetrafluorethyl-N,N-diethylamin, 1,1,2-Trifluor-2-(trifluormethyl)ethyl-N,N-dimethylamin, 1,1,2-Trifluor-2-(trifluormethyl)ethyl-N,N-diethylamin (Ishikawa-Reagenz), 1,1,2-Trifluor-2-chlor-ethyl-N,N-dimethylamin und 1,1,2-Trifluor-2-chlor-ethyl-N,N-diethylamin (Yarovenko-Reagenz), wobei 1,1,2,2-Tetrafluorethyl-N,N-dimethylamin und 1,1,2,2-Tetra-fluorethyl-N,N-diethylamin bevorzugt und 1,1,2,2-Tetrafluorethyl-N,N-dimethylamin besonders bevorzugt ist.

Es ist möglich Chloramine oder deren Salze (analog Vilsmeier Komplex) aus z.B Difluoroessigsäuredimethylamid und z. B Oxalylchlorid , Phosgen oder POCl3 herzustellen:

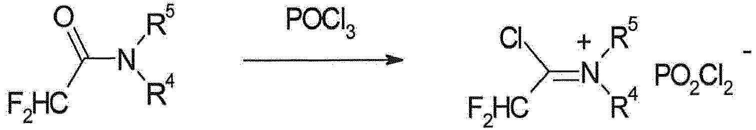

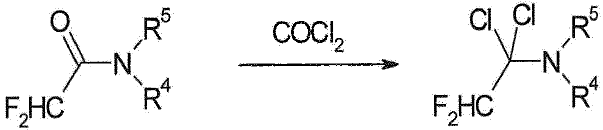

### Basen

Die Umsetzung der α,α-Dihalogenamine der Formel (III) mit den Carbonsäureestern der Formel (II) erfolgt üblicherweise in Gegenwart von Basen, durch die eine Deprotonierung der Carbonsäureester in α-Position zur Carbonyl-Gruppe erfolgt.

Gemäß der vorliegenden Erfindung sind daher alle Basen geeignet, die eine ausreichende Basizität zur Deprotonierung der Carbonsäureester aufweisen. Beispielsweise seien Alkalimetallhydroxide wie z.B. Lithium-, Natrium- oder Kaliumhydroxid, Alkalimetallalkoholate, wie z.B. NaOMe, NaOEt, NaOt-Bu, KOt-Bu, Hydride, wie z.B. NaH, KH, Alkyllithium-Reagenzien, wie z.B. n-BuLi oder t-Buli, Li N(iPr)₂, Grignard-Reagenzien, wie z.B. CH₃MgCl; Phosphazene und 1,8-Diazabicyclo[5.4.0]undecen (DBU) genannt.

Die Umsetzung der α,α-Dihalogenamine mit den Carbonsäureestern erfolgt üblicherweise bei Temperaturen von -50 bis 60 °C, vorzugsweise von -20 bis 40 °C, besonders bevorzugt von -10 bis 30 °C.

Die Reaktion kann unter vermindertem Druck, unter Normal- oder unter Hochdruck, vorzugsweise unter Normaldruck erfolgen.

Die Reaktion kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Fluorbenzol, Chlorbenzol oder Dichlorbenzol; Ethern, wie z.B. Diethylether, Diphenylether, Methyl-tert-butylether, Isopropylethylether, Dioxan, Diglym, Dimethylglycyl, Dimethoxyethane (DME) oder THF; Nitrilen wie Methylnitril, Butylnitril oder Phenylnitril; Amide wie Dimethylformamid (DMF) oder N-methylpyrrolidon (NMP) oder Mischungen solcher Lösungsmittel geeignet, wobei THF, DME, Diethylether besonders bevorzugt sind. Die Umsetzung kann in Überschuss von Carbonsäureester erfolgen (z.B in Ethylacetat).

Die Base und der Carbonsäureester werden vorzugsweise in äquimolaren Mengen eingesetzt. Alternativ kann die Base auch im Überschuss eingesetzt werden. Das Verhältnis von Base : Carbonsäureester liegt erfindungsgemäß zwischen 1.5:1 und 0.9:1, vorzugsweise zwischen 1.4:1, besonders bevorzugt zwischen 1.3:1 und 1.05:1.

In einer bevorzugten Ausführungsform des erfindungsgemäßen Verfahrens wird der Carbonsäureester in Substanz oder gelöst in einem geeigneten Lösungsmittel vorgelegt und sukzessive mit der Base versetzt und anschließend mit dem α,α-Dihalogenamin umsetzt.

Aufgrund der Hydrolyseempfindlichkeit der α,α-Dihalogenamine, ist die Umsetzung in wasserfreien Apparaturen unter Inertgasatmosphäre durchzuführen.

Die Erfindung soll anhand der folgenden Ausführungsbeispiele näher erläutert werden, ohne sie jedoch auf diese zu beschränken.

### Herstellungsbeispiele

### Beispiel 1: Herstellung von Difluoroacetessigsäureethylester

48 g Ethylacetat wurden vorgelegt und bei RT mit einer 10 %igen Lösung von 32.4 g Natriumethanolat in Ethanol versetzt. Das Gemisch wurde 1 h bei RT nachgerührt und darauf mit 36 g 1,1,2,2-Tetrafluorethyldimethylamin versetzt. Anschließend wurde die Lösung 2 h bei 30 °C gerührt, mit H₂O versetzt und auf pH 5 eingestellt. Nach Extraktion mit Ethylacetat und anschließender Destillation wurden 27 g (65 %) Difluoroacetessigsäureethylester (Siedepunkt 90-94°C/100 mbar) erhalten.

### Beispiel 2:

Analog zu Beispiel 1 wurde jedoch Natriumhydrid anstatt Natriumethanolat verwendet. Die erzielte Ausbeute beträgt 79 %.

### Beispiel 3.

Analog zu Beispiel 1 wurde jedoch 1,1-Dichlor-2,2-difluorethyldimethylamin (hergestellt aus Difluoressigsäuredimethylamid und Oxalylchlorid bei 80°C) verwendet. Die erzielte Ausbeute beträgt 63 %.

## Patentansprüche

1. Verfahren zur Herstellung von Verbindungen der Formel (I) 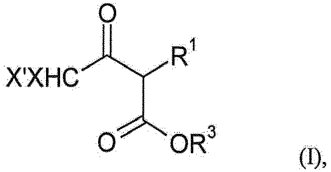 in der
X Fluor, Chlor oder CF₃ ist,
X' Fluor, Chlor oder Brom ist,
R¹ ausgewählt ist aus H, C₁₋₁₂-Alkyl-Resten, C₅₋₁₈-Aryl, Cl, Brom und Fluor und
R³ unabhängig von R¹ ausgewählt ist aus C₁₋₁₂-Alkyl-, C₅₋₁₈-Aryl- oder C₇₋₁₉-Aryl-alkyl-Resten,
durch Umsetzung von α,α-Dihalogenaminen der Formel (III) 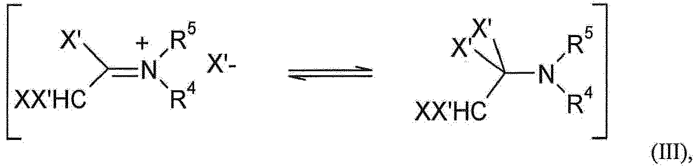 in welcher
R⁴ ausgewählt ist aus C₁₋₁₂-Alkyl-Resten, C₅₋₁₈-Aryl- oder C₇₋₁₉-Arylalkyl-Resten,
R⁵ unabhängig von R⁴ ausgewählt ist aus C₁₋₁₂-Alkyl-Resten, C₅₋₁₈-Aryl- oder C₇₋₁₉-Arylalkyl-Resten,
mit Carbonsäureestern der Formel (II) 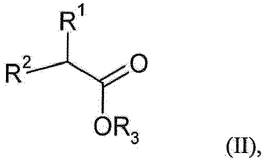 in welcher
R ² ausgewählt ist aus H, C₁₋₁₂-Alkyl-Resten, C₅₋₁₈-Aryl Cl, Brom und Fluor.

2. Verfahren gemäß Anspruch 1, **dadurch gekennzeichnet, dass**
X ausgewählt ist aus Fluor, Chlor oder CF₃;
X' ausgewählt ist aus Fluor oder Cl;
R¹ ausgewählt ist aus C₁₋₄-Alkyl-Resten;
R² unabhängig von R¹ ausgewählt ist aus C₁₋₄-Alkyl-Resten;
R³ unabhängig von R¹ ausgewählt ist aus C₁₋₄-Alkyl;
R⁴ ausgewählt ist aus C₁₋₄-Alkyl-Resten und
R⁵ unabhängig von R⁴ ausgewählt ist aus C₁₋₄-Alkyl-Resten.

3. Verfahren gemäß einem der Ansprüche 1 oder 2, **dadurch gekennzeichnet, dass**
X ausgewählt ist aus Fluor oder Cl;
X' Fluor ist;
R¹ ausgewählt ist aus Wasserstoff oder Fluor;
R² Wasserstoff ist;
R³ ausgewählt ist aus Methyl oder Ethyl;
R⁴ ausgewählt ist aus Methyl oder Ethyl;
R⁵ ausgewählt ist aus Methyl oder Ethyl.

4. Verfahren gemäß einem der Ansprüche 1 bis 3, **dadurch gekennzeichnet, dass** die Umsetzung in Gegenwart einer Base erfolgt.

5. Verfahren gemäß einem der Ansprüche 1 bis 4, **dadurch gekennzeichnet, dass** die α,α-Dihalogenamine ausgewählt sind aus der Gruppe bestehend aus 1,1,2,2-Tetrafluorethyl-N,N-dimethylamin, 1,1,2,2-Tetrafluorethyl-N,N-diethylamin, 1,1,2-Trifluor-2-(trifluormethyl)-ethyl-N,N-dimethylamin, 1,1,2-Trifluor-2-(trifluormethyl)ethyl-N,N-diethylamin (Ishikawa-Reagenz), 1,1,2-Trifluor-2-chlor-ethyl-N,N-dimethylamin und 1,1,2-Trifluor-2-chlorethyl-N,N-diethylamin (Yarovenko-Reagenz).

6. Verfahren gemäß einem der Ansprüche 1 bis 5, **dadurch gekennzeichnet, dass** die Base ausgewählt ist aus der Gruppe bestehend aus Alkalimetallhydroxiden, Alkalimetallalkoholaten, Hydriden, Alkyllithium-Reagenzien und Grignard-Reagenzien oder deren Mischungen.

7. Verfahren gemäß einem der Ansprüche 1 bis 6, **dadurch gekennzeichnet, dass** die Carbonsäureester ausgewählt sind aus Essigsäuremethylester, Essigsäureethylester, Fluoressigsäureethylester, Bromessigsäureethylester, Propionsäureethylester, Phenylessigsäureethylester und Benzoesäureethylester.

## Claims

1. Process for preparing compounds of the formula (I) 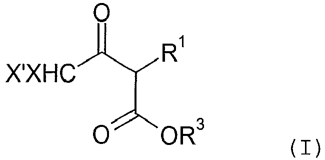 in which
X is fluorine, chlorine or CF₃,
X' is fluorine, chlorine or bromine,
R¹ is selected from H, C₁₋₁₂-alkyl radicals, C₅₋₁₈-aryl, Cl, bromine and fluorine and
R³, independently of R¹, is selected from C₁₋₁₂-alkyl, C₅₋₁₈-aryl or C₇₋₁₉-arylalkyl radicals,
by reacting α,α-dihaloamines of the formula (III) 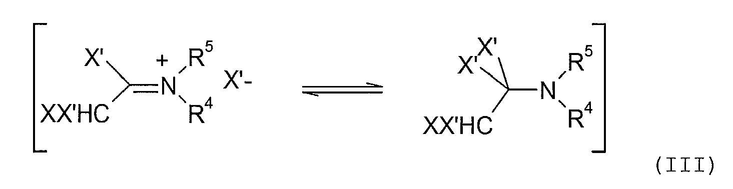 in which
R⁴ is selected from C₁₋₁₂-alkyl radicals, C₅₋₁₈-aryl radicals or C₇₋₁₉-arylalkyl radicals,
R⁵, independently of R⁴, is selected from C₁₋₁₂-alkyl radicals, C₅₋₁₈-aryl radicals or C₇₋₁₉-arylalkyl radicals,
with carboxylic esters of the formula (II) 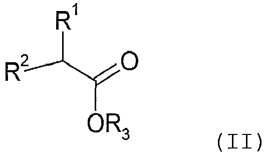 in which
R ² is selected from H, C₁₋₁₂-alkyl radicals, C₅₋₁₈-aryl, Cl, bromine and fluorine.

2. Process according to Claim 1, **characterized in that**
X is selected from fluorine, chlorine or CF₃;
X' is selected from fluorine or Cl;
R¹ is selected from C₁₋₄-alkyl radicals;
R², independently of R¹, is selected from C₁₋₄-alkyl radicals;
R³, independently of R¹, is selected from C₁₋₄-alkyl;
R⁴ is selected from C₁₋₄-alkyl radicals and
R⁵, independently of R⁴, is selected from C₁₋₄-alkyl radicals.

3. Process according to one of Claims 1 and 2, **characterized in that**
X is selected from fluorine or chlorine;
X' is fluorine;
R¹ is selected from hydrogen or fluorine;
R² is hydrogen;
R³ is selected from methyl or ethyl;
R⁴ is selected from methyl or ethyl;
R⁵ is selected from methyl or ethyl.

4. Process according to one of Claims 1 to 3, **characterized in that** the reaction is effected in the presence of a base.

5. Process according to one of Claims 1 to 4, **characterized in that** the α,α-dihaloamines are selected from the group consisting of 1,1,2,2-tetrafluoroethyl-N,N-dimethylamine, 1,1,2,2-tetrafluoroethyl-N,N-diethylamine, 1,1,2-trifluoro-2-(trifluoromethyl)ethyl-N,N-dimethylamine, 1,1,2-trifluoro-2-(trifluoromethyl)ethyl-N,N-diethylamine (Ishikawa reagent), 1,1,2-trifluoro-2-chloroethyl-N,N-dimethylamine and 1,1,2-trifluoro-2-chloroethyl-N,N-diethylamine (Yarovenko reagent).

6. Process according to one of Claims 1 to 5, **characterized in that** the base is selected from the group consisting of alkali metal hydroxides, alkali metal alkoxides, hydrides, alkyllithium reagents and Grignard reagents, or mixtures thereof.

7. Process according to one of Claims 1 to 6, **characterized in that** the carboxylic esters are selected from methyl acetate, ethyl acetate, ethyl fluoroacetate, ethyl bromoacetate, ethyl propionate, ethyl phenylacetate and ethyl benzoate.

## Revendications

1. Procédé pour la préparation de composés de formule (I) 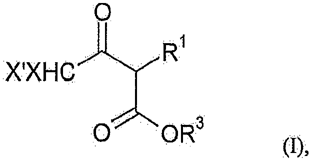 dans laquelle
X représente fluor, chlore ou CF₃ ;
X' représente fluor, chlore ou brome,
R¹ est choisi parmi H, les radicaux C₁-C₁₂-alkyle, C₅₋₁₈-aryle, Cl, brome et fluor et
R³ est choisi, indépendamment de R¹, parmi les radicaux C₁₋₁₂-alkyle, C₅₋₁₈-aryle ou C₇₋₁₉-arylalkyle
par transformation d'α,α-dihalogénoamines de formule (III) 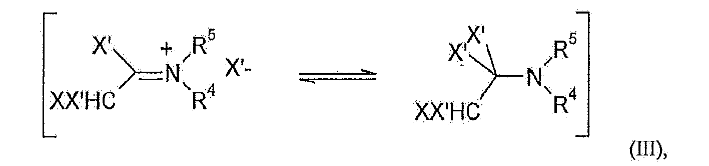 dans laquelle
R⁴ est choisi parmi les radicaux C₁₋₁₂-alkyle, C₅₋₁₈-aryle ou C₇₋₁₉-arylalkyle,
R⁵ est choisi, indépendamment de R⁴, parmi les radicaux C₁₋₁₂-alkyle, C₅₋₁₈-aryle ou C₇₋₁₉-arylalkyle,
avec des esters d'acide carboxylique de formule (II) 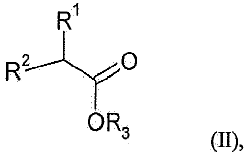 dans laquelle
R² est choisi parmi H, les radicaux C₁₋₁₂-alkyle, C₅₋₁₈-aryle, Cl, brome et fluor.

2. Procédé selon la revendication 1, **caractérisé en ce que**
X est choisi parmi fluor, chlore ou CF₃ ;
X' est choisi parmi fluor ou Cl ;
R¹ est choisi parmi les radicaux C₁₋₉-alkyle ;
R² est choisi, indépendamment de R¹, parmi les radicaux C₁₋₄-alkyle ;
R³ est choisi, indépendamment de R¹, parmi C₁₋₄-alkyle ;
R⁴ est choisi parmi les radicaux C₁₋₄-alkyle et
R⁵ est choisi, indépendamment de R⁴, parmi les radicaux C₁₋₉-alkyle.

3. Procédé selon l'une quelconque des revendications 1 ou 2, **caractérisé en ce que**
X est choisi parmi fluor ou chlore ;
X' représente fluor ;
R¹ est choisi parmi hydrogène ou fluor ;
R² représente hydrogène ;
R³ est choisi parmi méthyle ou éthyle ;
R⁴ est choisi parmi méthyle ou éthyle ;
R⁵ est choisi parmi méthyle ou éthyle.

4. Procédé selon l'une quelconque des revendications 1 à 3, **caractérisé en ce que** la transformation a lieu en présence d'une base.

5. Procédé selon l'une quelconque des revendications 1 à 4, **caractérisé en ce que** les α,α-dihalogénoamines sont choisies dans le groupe constitué par la 1,1,2,2-tétrafluoréthyl-N,N-diméthylamine, la 1,1,2,2-tétrafluoréthyl-N,N-diéthylamine, la 1,1,2-trifluoro-2-(trifluorométhyl)-éthyl-N,N-diméthylamine, la 1,1,2-trifluoro-2-(trifluorométhyl)éthyl-N,N-diéthylamine (réactif d'Ishikawa), la 1,1,2-trifluoro-2-chloréthyl-N,N-diméthylamine et la 1,1,2-trifluoro-2-chloréthyl-N,N-diéthylamine (réactif de Yarovenko).

6. Procédé selon l'une quelconque des revendications 1 à 5, **caractérisé en ce que** la base est choisie dans le groupe constitué par les hydroxydes de métal alcalin, les alcoolates de métal alcalin, les hydrures, les réactifs de type alkyllithium et les réactifs de Grignard ou leurs mélanges.

7. Procédé selon l'une quelconque des revendications 1 à 6, **caractérisé en ce que** les esters d'acide carboxylique sont choisis parmi l'ester méthylique de l'acide acétique, l'ester éthylique de l'acide acétique, l'ester éthylique de l'acide fluoroacétique, l'ester éthylique de l'acide bromoacétique, l'ester éthylique de l'acide propionique, l'ester éthylique de l'acide phénylacétique et l'ester éthylique de l'acide benzoïque.
